# EUROPEAN PATENT APPLICATION

(11) **EP 4 487 905 A1**
(43) Date of publication of application: **08.01.2025**
(21) Application number: 23760218.0
(22) Date of filing: 28.02.2023
(51) Int. Cl.: A61N 5/10

(54) **PARTICLE BEAM THERAPY FACILITY AND PARTICLE BEAM THERAPY DEVICE**

(30) Priority: 28.02.2022 JP 2022029191
(71) Applicant: Sumitomo Heavy Industries, Ltd., Tokyo 141-6025 (JP)
(72) Inventor: NAKAKITA Masaru, Niihama-shi, Ehime 792-8588 (JP); YOKOYAMA Kazuhiro, Niihama-shi, Ehime 792-8588 (JP)
(74) Representative: Louis Pöhlau Lohrentz
(86) International application number: PCT/JP2023/007355
(87) International publication number: WO 2023/163220

(57) **Abstract**

A particle beam therapy facility includes: a therapy room that is independent of other rooms and that is used to perform therapy with particle beams; a plurality of preparation rooms that is used to perform preparation for the therapy; and a transfer room that is independent of other rooms and that is used to transfer between the therapy room and the preparation rooms, in which the transfer room includes a single continuous space that allows communication between a therapy room entrance of the therapy room and a preparation room entrance of each of the plurality of preparation rooms.

## Description

### Technical Field

The present disclosure relates to a particle beam therapy facility and a particle beam therapy device.

### Background Art

Boron neutron capture therapy (BNCT) using a boron compound is known as neutron capture therapy of irradiating cancer cells with neutron rays to kill the cancer cells. In the boron neutron capture therapy, boron that has been incorporated into the cancer cells in advance is irradiated with neutron rays, thereby selectively destroying the cancer cells through the scattering of the resulting heavy charged particles.

As a device that generates neutron rays used for the aforementioned purpose, for example, a neutron ray generating device disclosed in PTL 1 is used. The neutron ray generating device disclosed in PTL 1 performs therapy by being disposed in front of an irradiation port of neutron rays while a patient is fixed to a placement member.

### Citation List

### Patent Literature

[PTL 1] Japanese Unexamined Patent Publication No. 2020-146119

### Summary of Invention

### Technical Problem

As mentioned above, in order to perform therapy with particle beams, preparations such as adjusting and fixing the patient's posture are performed in a preparation room before the therapy. Therefore, the preparation room is provided as a separate room from a therapy room. Here, in a case where a plurality of combinations of one preparation room for one therapy room are provided in order to perform preparations and therapy for a large number of patients, there is a problem that the particle beam therapy facility becomes large in size.

Therefore, an object of the present disclosure is to provide a particle beam therapy facility and a particle beam therapy device capable of downsizing the facility.

### Solution to Problem

According to the present disclosure, there is provided a particle beam therapy facility including: a therapy room that is independent of other rooms and that is used to perform therapy with particle beams; a plurality of preparation rooms that are used to perform preparations for the therapy; and a transfer room that is independent of other rooms and that is used to transfer between the therapy room and the preparation rooms, in which the transfer room includes a single continuous space that allows communication between a therapy room entrance of the therapy room and a preparation room entrance of each of the plurality of preparation rooms.

In the particle beam therapy facility according to the present disclosure, the plurality of preparation rooms are provided for a single independent therapy room. Therefore, when the preparation for therapy in the therapy room is being performed in a certain preparation room, a therapy simulation can be performed in another preparation room. Consequently, the facility can be downsized because a simulation room can be omitted. Here, the particle beam therapy facility includes a transfer room that is used to transfer between the therapy room and the preparation rooms. Additionally, the transfer room includes a single continuous space that allows communication between a therapy room entrance of the therapy room and a preparation room entrance of each of the plurality of preparation rooms. Therefore, the particle beam therapy facility can quickly perform transport through the common space of the transfer room, regardless of which preparation room the therapy target object is transported from. Accordingly, the layout between the therapy room and the plurality of preparation rooms can be made compact. As a result, the facility can be downsized.

In the particle beam therapy facility, a support portion provided in the transfer room; and a transport mechanism that is supported by the support portion to be movable between the plurality of preparation rooms and the therapy room and that transports a therapy target object between the plurality of preparation rooms and the therapy room may be further provided. In this case, the particle beam therapy facility can quickly perform transport by using the common transport mechanism supported by the support portion of the transfer room, regardless of which preparation room the therapy target object is transported from. Therefore, the layout between the therapy room and the plurality of preparation rooms can be made compact. As a result, the facility can be downsized.

The transport mechanism may be rotatable around a center axis extending in an up-down direction. Consequently, the transport mechanism can quickly perform transport by rotating and directing a transport arm toward the desired room.

The plurality of preparation rooms may be disposed to surround the transfer room. In this case, the number of preparation rooms that can be disposed with respect to the area of the transfer room can be increased.

A boron concentration measurement room that is independent of other rooms and that is used to measure a boron concentration of a therapy target object may be further provided. In this case, since the boron concentration of the therapy target object can be quickly measured, the number of personnel and time required for the measurement can be reduced.

According to the present disclosure, there is provided a particle beam therapy device including: a support portion provided between a therapy section for performing therapy with particle beams and a plurality of preparation sections for performing preparations for the therapy; and a transport mechanism that is supported by the support portion to be movable between the plurality of preparation sections and the therapy section and that transports a therapy target object between the plurality of preparation sections and the therapy section.

The particle beam therapy device according to the present disclosure includes the support portion provided between the therapy section and the plurality of preparation sections. In addition, the particle beam therapy device includes the transport mechanism that is supported by the support portion to be movable between the plurality of preparation sections and the therapy section and that transports the therapy target object between the plurality of preparation sections and the therapy section. Therefore, the particle beam therapy device can quickly perform transport by using the common transport mechanism supported by the support portion, regardless of which preparation section the therapy target object is transported from. Accordingly, the layout between the therapy section and the plurality of preparation sections can be made compact. As a result, the facility can be downsized.

In the particle beam therapy device, a therapy room that is independent of other rooms and that is provided with the therapy section; a plurality of preparation rooms that are each provided with the preparation section; and a transfer room that is independent of other rooms, that is provided with the transport mechanism, and that is used to transfer between the therapy room and the preparation rooms may be further provided. A plurality of preparation rooms are provided for a single independent therapy room. Therefore, when the preparation for therapy in the therapy room is being performed in a certain preparation room, the therapy simulation can be performed in another preparation room. Consequently, the facility can be downsized because the simulation room can be omitted. Here, the particle beam therapy device includes the transfer room that is used to transfer between the therapy room and the preparation rooms. In addition, the transfer room is provided with the transport mechanism that transports the therapy target object between the therapy room and the preparation room. Therefore, the particle beam therapy facility can quickly perform transport by using the common transport mechanism of the transfer room, regardless of which preparation room the therapy target object is transported from. Accordingly, the layout between the therapy room and the plurality of preparation rooms can be made compact. As a result, the facility can be downsized.

The plurality of preparation sections may be disposed to surround the transport mechanism. In this case, the number of preparation sections that can be disposed with respect to the area of the transfer room can be increased.

The transport mechanism may include a transport arm that is rotatable around a center axis extending in an up-down direction. Consequently, the transport mechanism can quickly perform transport by rotating and directing a transport arm toward the desired room.

The support portion may be provided between the therapy section, the plurality of preparation sections, and an imaging section for imaging the therapy target object, and the transport mechanism may be supported by the support portion to be movable between the plurality of preparation sections, the imaging section, and the therapy section and may transport the therapy target object between the plurality of preparation sections and the imaging section, and the therapy section. In this case, the particle beam therapy device can quickly check a fixation position of the therapy target object by performing the therapy simulation in a certain preparation section to determine the fixation position, and then transporting the therapy target object to the imaging section for imaging while keeping the therapy target object fixed. Additionally, since the imaging section is provided near the preparation section, the preparation immediately before the therapy can be performed in the same preparation section as the preparation section where the simulation has been performed. In this case, since there is no need to correct alignment errors between the simulation and the actual therapy preparation, the preparation time immediately before the therapy can be shortened.

In the particle beam therapy device, an imaging room that is independent of other rooms and that is provided with the imaging section may be further provided, and the imaging room may enable imaging of the therapy target object prepared in each of the plurality of preparation rooms that are each provided with the preparation section. In this case, the particle beam therapy device can quickly check the fixation position of the therapy target object by performing the therapy simulation in a certain preparation room to determine the fixation position, and then transporting the therapy target object to the imaging room for imaging while keeping the therapy target object fixed. Additionally, since the imaging room is provided near the preparation room, the preparation immediately before the therapy can be performed in the same preparation room as the preparation room where the simulation has been performed. In this case, since there is no need to correct alignment errors between the simulation and the actual therapy preparation, the preparation time immediately before the therapy can be shortened.

The transport mechanism may transport a first therapy target object from a first preparation section to the therapy section and may transport, during therapy of the first therapy target object in the therapy section, a second therapy target object from a second preparation section to a plurality of other preparation sections excluding the therapy section or to an imaging section for imaging the therapy target object. In this case, it is possible to proceed with the therapy preparation for the second therapy target object in another preparation section simultaneously with the therapy for the first therapy target object.

The transport mechanism may transport a first therapy target object from a first preparation section to an imaging section for imaging the therapy target object and may transport, during imaging of the first therapy target object in the imaging section or during preparation of the first therapy target object in the first preparation section, a second therapy target object from a second preparation section to the therapy section. In this case, it is possible to proceed with therapy of another second therapy target object in another preparation room simultaneously with the simulation of the first therapy target object or the imaging for checking the simulation.

### Advantageous Effects of Invention

According to the present disclosure, it is possible to provide a particle beam therapy facility and a particle beam therapy device capable of downsizing the facility.

### Brief Description of Drawings

Fig. 1 is a schematic view showing a particle beam therapy facility and a particle beam therapy device according to an embodiment of the present disclosure.
Fig. 2 is a schematic view showing a neutron ray generating device.
Fig. 3 is a schematic view showing a particle beam therapy facility according to a comparative example.
Fig. 4 is a diagram showing an operation timing of the particle beam therapy facility.
Fig. 5 is a diagram showing an operation timing of the particle beam therapy facility according to the comparative example.
Fig. 6 is a diagram showing an operation timing of a particle beam therapy facility according to an example.
Fig. 7 is a table comparing conditions of the comparative example and the example.
Figs. 8A and 8B are schematic views showing particle beam therapy facilities according to modification examples.

### Description of Embodiments

Hereinafter, a preferred embodiment of the present disclosure will be described in detail with reference to the drawings.

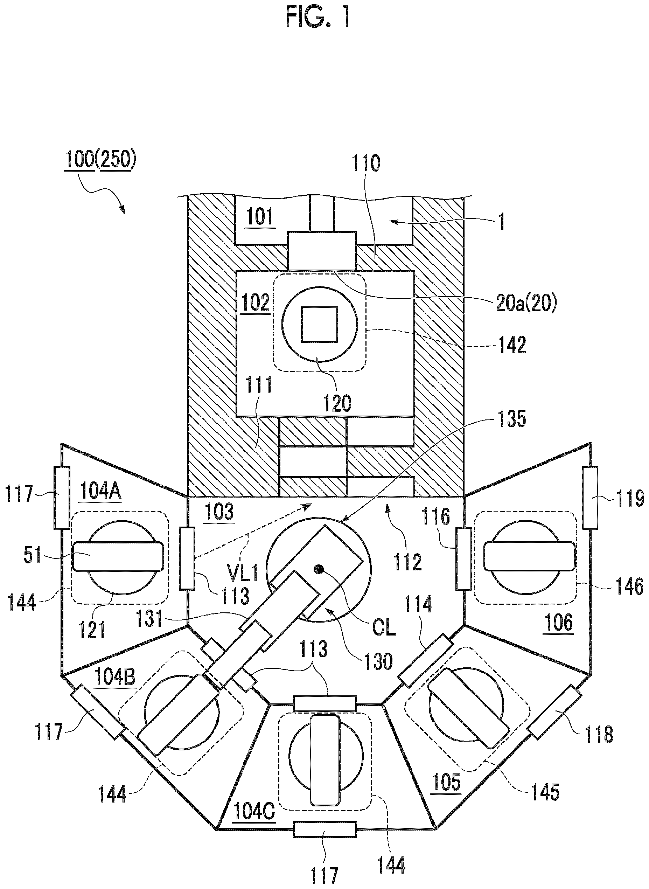

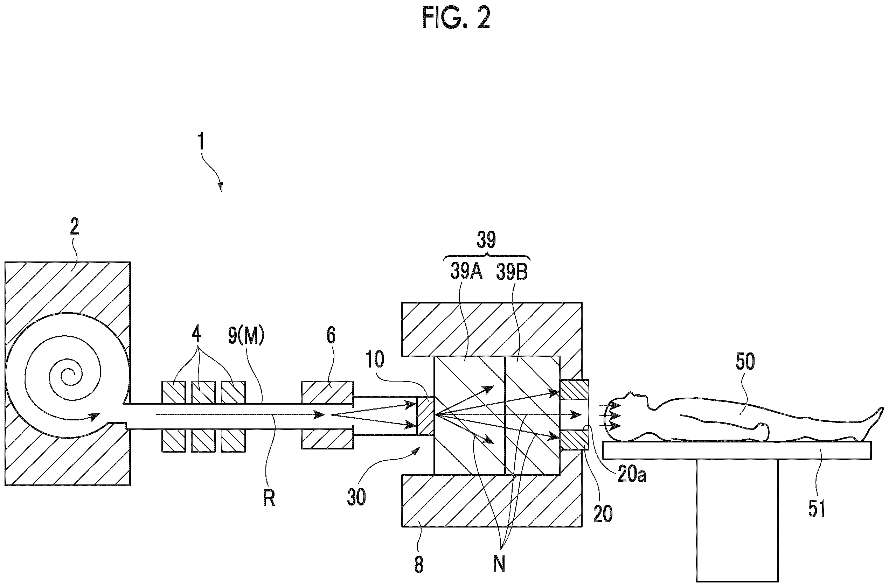

Fig. 1 is a schematic view showing a particle beam therapy facility 100 and a particle beam therapy device 250 according to an embodiment of the present disclosure. The particle beam therapy facility 100 is a facility that performs therapy by using neutron rays generated by a neutron ray generating device 1 as particle beams. The particle beam therapy device 250 is a device that performs therapy by using neutron rays generated by the neutron ray generating device 1 as particle beams. The neutron ray generating device 1 is used as a neutron capture therapy apparatus that performs cancer therapy using boron neutron capture therapy (BNCT). First, the configuration of the neutron ray generating device 1 that functions as the neutron capture therapy apparatus will be described with reference to Fig. 2. The neutron ray generating device 1 irradiates, for example, a tumor of a patient 50 (therapy target object), to which boron (¹⁰B) has been administered, with neutron rays N.

The neutron ray generating device 1 includes an accelerator 2. The accelerator 2 accelerates particles to emit a particle beam R. For example, a cyclotron, a linear accelerator, or the like may be employed as the accelerator 2.

The particle beam R emitted from the accelerator 2 passes through a transport path 9, referred to as a beam duct, whose inside is maintained in a vacuum and allows the beam to pass through, and is transported to a target disposition portion 30. The target disposition portion 30 is a portion where a target 10 is disposed, and includes a mechanism that holds the target 10 in the posture for irradiation. In the target disposition portion 30, the target 10 is disposed at a position facing an end portion (emission port) of the transport path 9. The particle beam R emitted from the accelerator 2 passes through the transport path 9 and proceeds toward the target 10 disposed at the end portion of the transport path 9. A plurality of electromagnets 4 (such as quadrupole electromagnets) and a scanning electromagnet 6 are provided along the transport path 9. The plurality of electromagnets 4 perform, for example, beam axis adjustment and the like of the particle beam R by using electromagnets.

The scanning electromagnet 6 performs a scan with the particle beam R and performs irradiation control of the particle beam R onto the target 10. The scanning electromagnet 6 controls the irradiation position of the particle beam R on the target 10.

The neutron ray generating device 1 generates the neutron rays N by irradiating the target 10 with the particle beam R and emits the neutron rays N toward the patient 50. The neutron ray generating device 1 includes the target 10, a shield member 8, a deceleration member 39, and a collimator 20.

The target 10 generates the neutron rays N when irradiated with the particle beam R. The target 10 is a solid member formed of a material that generates the neutron rays N when irradiated with the particle beam R. Specifically, the target 10 is formed of, for example, beryllium (Be), lithium (Li), tantalum (Ta), or tungsten (W) and has, for example, a solid disc shape with a diameter of 160 mm. The target 10 is not limited to a disc shape and may have other shapes.

The deceleration member 39 decelerates the neutron rays N generated by the target 10 (reduces the energy of the neutron rays N). The deceleration member 39 may have a laminated structure consisting of a layer 39A that primarily decelerates fast neutrons contained in the neutron rays N and a layer 39B that primarily decelerates epithermal neutrons contained in the neutron rays N.

The shield member 8 blocks the generated neutron rays N, gamma rays generated as a result of generating the neutron rays N, and the like to prevent their release to the outside. The shield member 8 is provided to surround the deceleration member 39. An upper portion and a lower portion of the shield member 8 extend to an upstream side of the particle beam R with respect to the deceleration member 39.

The collimator 20 shapes the irradiation field of the neutron rays N and includes an irradiation port 20a through which the neutron rays N pass. The collimator 20 is, for example, a block-shaped member including the irradiation port 20a at its center.

Next, the configuration of the particle beam therapy facility 100 will be described with reference to Fig. 1. As shown in Fig. 1, the particle beam therapy facility 100 includes an accelerator room 101, a therapy room 102, a transfer room 103, a plurality of preparation rooms 104A, 104B, and 104C, an imaging room 105, and a boron concentration measurement room 106. The accelerator room 101, the therapy room 102, the transfer room 103, each of the preparation rooms 104A, 104B, and 104C, the imaging room 105, and the boron concentration measurement room 106 are rooms independent of other rooms. An independent room is a section that is formed as a single continuous space by being partitioned from other rooms by walls.

The accelerator room 101 is a room for primarily disposing the accelerator 2 and the transport path 9 of the aforementioned neutron ray generating device 1. The therapy room 102 is a room for administering therapy to the patient 50 by irradiating the patient 50 with neutron rays. The accelerator room 101 and the therapy room 102 are spaces partitioned by blockade walls such as concrete. The accelerator room 101 and the therapy room 102 are separated by a partition wall 110 of a building. The aforementioned collimator 20 is provided on a wall surface of the partition wall 110 on a therapy room 102 side.

In the therapy room 102, a therapy section 142 for performing therapy with neutron rays is provided in front of the collimator 20. The therapy section 142 includes, for example, various mechanisms required for therapy, such as a therapy table 120, which is provided in the vicinity of an outlet of the irradiation port 20a and is movable in XYZ directions with a placement member 51 placed thereon. The therapy table 120 is a mechanism for receiving the placement member 51, to which the patient 50 is fixed, and for disposing an affected part of the patient 50 in the vicinity of the irradiation port 20a through movement in the XYZ directions (including rotational movement). The therapy table 120 is liftable, lowerable, and slidable in each of the XYZ directions, is rotatable 360° around a Z axis, and is rotatable around an X axis and a Y axis by a predetermined angle. The therapy table 120 may be a fixed table that does not move. The therapy table 120 includes a single therapy room entrance 112 that is provided on a partition wall 111 on a side opposite to the accelerator room 101 among the blockade walls surrounding the therapy room 102 and that connects the therapy room 102 and the transfer room 103.

The transfer room 103 is a room for transferring between the therapy room 102, the preparation rooms 104A, 104B, and 104C, the imaging room 105, and the boron concentration measurement room 106. The transfer room 103 is a room that is separated from the therapy room 102 by the partition wall 111 in a radiation-shielded state and that spreads to a side in front of the therapy room entrance 112 of the therapy room 102. The preparation rooms 104A, 104B, and 104C, the imaging room 105, and the boron concentration measurement room 106 are disposed to surround the transfer room 103. The preparation rooms 104A, 104B, and 104C, the imaging room 105, and the boron concentration measurement room 106 are disposed in an arch shape in this order from one end portion to the other end portion in a width direction of the partition wall 111.

The preparation rooms 104A, 104B, and 104C include preparation room entrances 113 that are connected to the transfer room 103. The imaging room 105 includes an imaging room entrance 114 that is connected to the transfer room 103. The boron concentration measurement room 106 includes a measurement room entrance 116 that is connected to the transfer room 103. As a result, the transfer room 103 includes a single continuous space that allows communication between the therapy room entrance 112 of the therapy room 102, the preparation room entrance 113 of each of the plurality of preparation rooms 104A, 104B, and 104C, the imaging room entrance 114 of the imaging room 105, and the measurement room entrance 116 of the boron concentration measurement room 106.

The single continuous space that couples the entrances 112, 113, 114, and 116 to each other is a space having a sufficient width and shape to allow the placement member 51 to be transported between the rooms by the same transport mechanism. In the space, when viewed from the preparation room entrance 113 toward the therapy room entrance 112 (for example, along a line of sight VL1), the line of sight is not obstructed by structures that partition the space, such as walls or staircases of the building. In the configuration shown in Fig. 1, when viewed from the preparation room entrance 113 of the preparation room 104C toward the therapy room entrance 112, the line of sight is obstructed by the transport mechanism, but the line of sight is not obstructed by structures that partition the space. Therefore, it can be said that the preparation room entrance 113 of the preparation room 104C and the therapy room entrance 112 communicate with each other through the single continuous space.

The preparation rooms 104A, 104B, and 104C include entrances 117 on a side opposite to the transfer room 103 to allow access from corridors, rooms, and the like on the side opposite to the transfer room 103. The imaging room 105 includes an entrance 118 on the side opposite to the transfer room 103 to allow access from the corridors, the rooms, and the like on the side opposite to the transfer room 103. The boron concentration measurement room 106 includes an entrance 119 on the side opposite to the transfer room 103 to allow access from the corridors, the rooms, and the like on the side opposite to the transfer room 103.

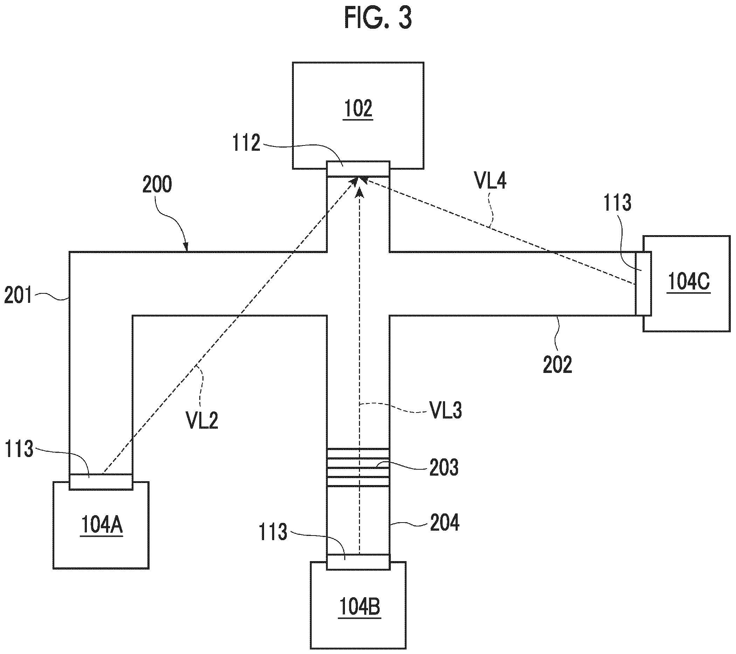

Fig. 3 shows a facility in which a long corridor 200 is provided between the plurality of preparation rooms 104A, 104B, and 104C and the therapy room 102. The preparation rooms 104A and 104C are connected to the therapy room 102 through curved corridors 201 and 202. The preparation room 104B is connected to the therapy room 102 through a corridor 204 including a staircase 203. Lines of sight VL2 and VL4 when viewed from the preparation room entrances 113 of the preparation rooms 104A and 104C toward the therapy room entrance 112 are obstructed by walls of the building. A line of sight VL3 when viewed from the preparation room entrance 113 of preparation room 104B toward the therapy room 102 is obstructed by the staircase 203 of the building. Therefore, the facility shown in Fig. 3 does not include a transfer room including a single continuous space that allows communication between the therapy room entrance 112 of the therapy room 102 and the preparation room entrance 113 of each of the plurality of preparation rooms 104A, 104B, and 104C.

The preparation rooms 104A, 104B, and 104C are rooms for performing preparations for therapy in the therapy room 102. The preparation rooms 104A, 104B, and 104C are provided with preparation sections 144 for performing preparations for therapy. The preparation section 144 includes, for example, a table 121 on which the placement member 51 is placed, a device used for alignment, and the like. The table 121 is used as one component of a positioning device for the patient 50 and is movable in the XYZ directions (including rotational movement), with the placement member 51 on which the patient 50 is fixed placed thereon. The table 121 is liftable, lowerable, and slidable in each of the XYZ directions, is rotatable 360° around the Z axis, and is rotatable around the X axis and the Y axis by a predetermined angle. In the preparation section 144, the state of the patient 50 in the therapy room 102 can be simulated through the alignment using the placement member 51, the table 121, and a laser and/or X-ray imaging device. Specifically, in the preparation section 144, the positions of the patient 50 and the irradiation port 20a in the therapy room 102 relative to the placement member 51 can be simulated.

Here, the preparations for the therapy in preparation rooms 104A, 104B, and 104C will be described. Since the neutron rays N used for therapy are diffusely released from the irradiation port 20a, the patient 50 is placed as close as possible to the irradiation port 20a. Meanwhile, a human body has a complex shape (particularly the neck). Currently, since there is no function of changing a beam irradiation direction, such as a gantry, the posture of the patient 50 is determined with respect to the fixed irradiation port 20a. Therefore, in a case of performing therapy by using the neutron ray generating device 1, the posture of the patient 50 on the placement member 51 is determined in advance. In particular, the patient 50 may be forced into an unnatural posture such that the patient 50 approaches the irradiation port 20a, and then, the patient 50 may be fixed to the placement member 51 by using a fixture. In order to perform these therapy preparations, the preparation rooms 104A, 104B, and 104C are provided. In addition, therapy simulations are also performed in the preparation rooms 104A, 104B, and 104C. The simulation is tasks such as examining the fixing method of the patient 50 and creating a fixing jig for the fixation determined after the examination. The simulation is performed a few days before the actual therapy.

The imaging room 105 is a room for imaging the patient 50. The imaging room 105 includes an imaging section 145. The imaging section 145 includes the table 121 and the X-ray imaging device. In the imaging section 145, the patient 50 is imaged by, for example, using the X-ray imaging device in order to check a positioning result of the patient 50 in the preparation section 144. The X imaging device includes a space for disposing the patient 50 between an X-ray irradiation device and an X-ray detection device (imaging plate (photographic negative)), and imaging is performed with the patient 50 interposed therebetween. The imaging section 145 may have a combination of the table 121 moving in the up-down direction and the traveling X-ray imaging device, or may have a combination of the table 121 moving up and down and back and forth and the rotating X-ray imaging device. The imaging room 105 enables imaging of the patient 50 prepared in each of the plurality of preparation rooms 104A, 104B, and 104C. In the preparation rooms 104A, 104B, and 104C, the therapy simulations are performed, and the adjustment of the fixation position of the patient 50 and the like are performed. In the imaging room 105, the patient 50 is transported while remaining fixed, and imaging is performed to check whether the fixation position is correct.

The boron concentration measurement room 106 is a room for measuring the boron concentration of the patient 50. The boron concentration measurement room 106 includes a boron concentration measurement section 146 for measuring the boron concentration. The boron concentration measurement section 146 includes a measurement device that measures the boron concentration of the patient 50. In the boron concentration measurement section 146, the boron concentration of the patient 50 prepared in each of the plurality of preparation rooms 104A, 104B, and 104C can be measured. In the boron concentration measurement section 146, blood collected from the patient 50 need only be provided. Therefore, the boron concentration measurement section 146 may or may not include the table 121. Even in a case where the boron concentration measurement section 146 includes the table 121, a moving mechanism or a rotating mechanism is not required. The boron concentration measurement room 106 may be omitted.

The transfer room 103 is provided with a support portion 135. The support portion 135 is a structure provided between the therapy section 142 and the plurality of preparation sections 144. The support portion 135 is fixed on a floor surface of the transfer room 103. The support portion 135 supports a transport mechanism 130. The transport mechanism 130 is supported by the support portion 135 to be movable between the plurality of preparation sections 144 and the therapy section 142 and transports the patient 50 between the plurality of preparation sections 144 and the therapy section 142. That is, the transport mechanism 130 transports the patient 50 between the therapy room 102 and the preparation rooms 104A, 104B, and 104C.

The transport mechanism 130 includes a transport arm 131 that is rotatable around a center axis CL extending in the up-down direction. The transport mechanism 130 rotates around the center axis CL and directs the tip of the transport arm 131 toward the desired room. Then, the transport arm 131 of the transport mechanism 130 enters the room through the entrance. The transport arm 131 holds the patient 50 along with the placement member 51 from inside the room and extracts the patient 50 from the room. Next, the transport arm 131 rotates around the center axis CL and faces the direction of the room of the transport destination. Then, the transport arm 131 enters the room together with the placement member 51 through the entrance. The transport arm 131 installs the patient 50 along with the placement member 51 in the room of the transport destination.

For example, when the patient 50 is fixed to the placement member 51 in the preparation room 104A for the therapy simulation of the patient 50, the transport mechanism 130 takes out the patient 50 along with the placement member 51 from the preparation room 104A and transports the patient 50 to the imaging room 105. Additionally, when the patient 50 is fixed to the placement member 51 in the preparation room 104A as the preparation for the therapy, the transport mechanism 130 takes out the patient 50 along with the placement member 51 from the preparation room 104A and transports the patient 50 to the therapy room 102.

Further, the transport mechanism 130 transports a certain patient 50 from the preparation room 104A to the therapy room 102. Then, the transport mechanism 130 transports, during the therapy of the patient 50 in the therapy room 102, another patient 50 from another preparation room 104B (or preparation room 104C) to another room excluding the therapy room 102. The other room is the imaging room 105, the boron concentration measurement room 106, or the like.

In addition, the transport mechanism 130 transports a certain patient 50 from the preparation room 104A to the imaging room 105. The transport mechanism 130 transports, during the imaging of the patient 50 in the imaging room 105 or during the imaging preparation in the preparation room 104A, another patient 50 from another preparation room 104B (or preparation room 104C) to the therapy room 102.

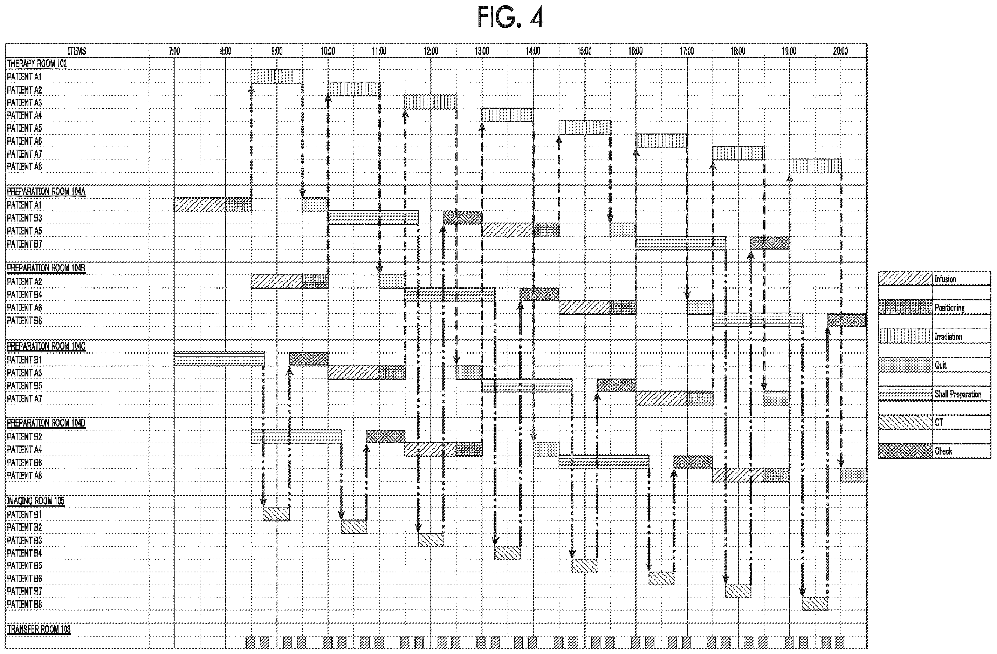

Next, an example of the operation of the particle beam therapy facility 100 will be described with reference to Fig. 4. Fig. 4 is a diagram showing the operation timing of the particle beam therapy facility 100 throughout a day. In Fig. 4, it is assumed that there are four preparation rooms, from the "preparation room 104A" to a "preparation room 104D", as the preparation rooms. Here, it is assumed that the "preparation room 104D" is added to the particle beam therapy facility 100 in Fig. 1. Further, it is assumed that the therapy of eight patients 50, from a "patient A1" to a "patient A8", is administered in the therapy room 102, and the therapy simulations of eight patients 50, from a "patient B1" to a "patient B8", are performed. In Fig. 4, the operation timing in each room of the particle beam therapy facility 100 is indicated by color-coded bars with patterns. Among these, "Infusion" means injecting a boron drug into the patient 50. "Positioning" means performing the task of positioning the posture of the patient 50 on the placement member 51. "Irradiation" means performing the therapy through irradiation with neutron rays. "Quit" means performing post-therapy treatments. "Shell Preparation" means performing a therapy simulation and preparing a fixing jig. "CT" means imaging the patient 50 by using a CT device. "Check" means checking the position of the patient 50 from the captured image.

In the therapy room 102, therapy for the eight patients, the "patient A1" to the "patient A8", is performed. In the imaging room 105, the eight patients, the "patient B1" to the "patient B8", are imaged. The "patient A1" to the "patient A8" and the "patient B1" to the "patient B8" are different individuals. In addition, it is assumed that the therapy simulations of the "patient A1" to the "patient A8" are completed by the previous day. It is assumed that the therapy of the "patient B1" to the "patient B8" will be performed at a later date. In the preparation room 104A, preparations and the like (preparations for therapy or simulations for creating the fixing jig) for the "patient A1", the "patient B2", the "patient A5", and the "patient B7" are performed. In the preparation room 104B, preparations and the like for the "patient A2", the "patient B4", the "patient A6", and the "patient B8" are performed. In the preparation room 104C, preparations and the like for the "patient B1", the "patient A3", the "patient B5", and the "patient A7" are performed. In the preparation room 104D, preparations and the like for the "patient B2", the "patient A4", the "patient B6", and the "patient A8" are performed. In Fig. 4, the timing at which the patient 50 is transported to another room is indicated by an arrow. Further, in the item for the transfer room 103, the timing during transport is indicated by a bar.

First, the therapy preparation for the "patient A1" is performed in the preparation room 104A, and after the preparation, the "patient A1" is transported to the therapy room 102 for the therapy and is then transported to the preparation room 104A for post-treatments. Simultaneously, the simulation preparation for the "patient B1" is performed in the preparation room 104C, and after the preparation, the "patient B1" is transported to imaging room 105 for imaging and is then transported to the preparation room 104C for checking. The transport mechanism 130 transports, during the therapy of the "patient A1" in the therapy room 102, another "patient B1" from the preparation room 104C, which is different from the preparation room 104A, to the imaging room 105, which is another room excluding the therapy room 102. Alternatively, the transport mechanism 130 transports, during the imaging preparation for the "patient B1" in the preparation room 104C, another "patient A1" from another preparation room 104A to the therapy room 102.

After a predetermined time has elapsed from the start of the preparation for the "patient A1" in the preparation room 104A, the preparation for the therapy of the "patient A2" starts in the preparation room 104B. The start of the preparation in the preparation room 104B is adjusted such that the therapy timing in the therapy room 102 for the "patient A2" does not overlap with the therapy timing in the therapy room 102 for the "patient A1". Meanwhile, after a predetermined time has elapsed from the start of the simulation in the preparation room 104C for the "patient B1", the preparation for the simulation of the "patient B2" starts in the preparation room 104D. The start of the preparation in the preparation room 104D is adjusted such that the imaging timing in the imaging room 105 for the "patient B2" does not overlap with the imaging timing in the imaging room 105 for the "patient B1".

Subsequently, in a similar manner, the preparation for the therapy and the therapy are performed in the order of the "patient A3", the "patient A4", ..., and the preparation for the simulation and the imaging are performed in the order of the "patient B3", the "patient B4" ....

In the example shown in Fig. 4, the imaging of the patients 50 (patients A1 to A8) for whom therapy is performed in the therapy room 102 is completed by the previous day. However, after the patient 50 is imaged in the imaging room 105, the preparation (injection) for the therapy and the therapy in the therapy room 102 may be performed on the same day. In this case, the patient 50 is imaged in the imaging room 105 while remaining fixed, for the final check of the patient's position immediately before the injection.

Next, the operations and effects of the particle beam therapy facility 100 according to the present embodiment will be described.

In the particle beam therapy facility 100 according to the present embodiment, the plurality of preparation rooms 104 are provided for a single independent therapy room 102. Therefore, when the preparation for the therapy in the therapy room 102 is being performed in a certain preparation room 104, the therapy simulation can be performed in another preparation room 104. Consequently, the facility can be downsized because the simulation room can be omitted. Here, the particle beam therapy facility 100 includes the transfer room 103 for transferring between the therapy room 102 and the preparation rooms 104. Additionally, the transfer room 103 includes a single continuous space that allows communication between the therapy room entrance 112 of the therapy room 102 and the preparation room entrance 113 of each of the plurality of preparation rooms 104. Therefore, the particle beam therapy facility 100 can quickly perform transport through the common space of the transfer room 103, regardless of which preparation room 104 the patient 50 is transported from. Accordingly, the layout between the therapy room 102 and the plurality of preparation rooms 104 can be made compact. As a result, the facility can be downsized.

In the particle beam therapy facility 100 according to the present embodiment, the plurality of preparation rooms 104 are provided for a single independent therapy room 102. Therefore, when the preparation for the therapy in the therapy room 102 is being performed in a certain preparation room 104, the therapy simulation can be performed in another preparation room 104. Consequently, the facility can be downsized because the simulation room can be omitted. Here, the particle beam therapy facility 100 includes the transfer room 103 for transferring between the therapy room 102 and the preparation rooms 104. In addition, the particle beam therapy facility 100 may further include the support portion 135 provided in the transfer room 103, and the transport mechanism 130 that is supported by the support portion 135 to be movable between the plurality of preparation rooms 104 and the therapy room 102 and that transports the patient 50 between the plurality of preparation rooms 104 and the therapy room 102. Therefore, the particle beam therapy facility 100 can quickly perform transport by using the common transport mechanism 130 supported by the support portion 135 of the transfer room 103, regardless of which preparation room 104 the patient 50 is transported from. Accordingly, the layout between the therapy room 102 and the plurality of preparation rooms 104 can be made compact. As a result, the facility can be downsized.

In the particle beam therapy facility 100, the transport mechanism 130 may be rotatable around the center axis extending in the up-down direction. Consequently, the transport mechanism 130 can quickly perform transport by rotating and directing the transport arm 131 toward the desired room.

In the particle beam therapy facility 100, the plurality of preparation rooms 104 may be disposed to surround the transfer room 103. In this case, the number of preparation rooms 104 that can be disposed with respect to the area of the transfer room 103 can be increased.

In the particle beam therapy facility 100, the boron concentration measurement room 106 that is independent of other rooms and is used to measure the boron concentration of the patient 50 may be further provided. In this case, since the boron concentration can be quickly measured by collecting blood from the patient 50, the number of personnel and the time required for the measurement can be reduced. That is, the neutron ray generating device 1 adjusts the amount of irradiation charge based on the measurement result of the boron concentration in the collected blood. In the boron concentration measurement room 106, the measurement result of the boron concentration in the blood can be quickly obtained. In a case where the boron concentration measurement room 106 includes a device that measures the boron concentration through image diagnosis, the measurement can be performed simply by transporting the patient 50 without collecting blood. The boron concentration measurement room 106 may be omitted by disposing such a device in the imaging room 105.

The particle beam therapy device 250 according to the present embodiment includes the support portion 135 between the therapy section 142 and the plurality of preparation sections 144. In addition, the particle beam therapy device 250 includes the transport mechanism 130 that is supported by the support portion 135 to be movable between the plurality of preparation sections 144 and the therapy section 142 and that transports the patient 50 between the plurality of preparation sections 144 and the therapy section 142. Therefore, the particle beam therapy device 250 can quickly perform transport by using the common transport mechanism 130 supported by the support portion 135, regardless of which preparation section 144 the patient 50 is transported from. Accordingly, the layout between the therapy section 142 and the plurality of preparation sections 144 can be made compact. As a result, the facility can be downsized.

In the particle beam therapy device 250, the plurality of preparation sections 144 may be disposed to surround the transfer room 103. In this case, the number of preparation sections 144 that can be disposed with respect to the area of the transfer room 103 can be increased.

In the particle beam therapy device 250, the transport mechanism 130 that transports the patient 50 between the therapy room 102 and the preparation rooms 104 may include the transport arm 131 that is rotatable around the center axis CL extending in the up-down direction. Consequently, the transport mechanism 130 can quickly perform transport by rotating and directing the transport arm 131 toward the desired room.

In the particle beam therapy device 250, the support portion 135 may be provided between the therapy section 142, the plurality of preparation sections 144, and the imaging section 145 for imaging the patient 50, and the transport mechanism 130 may be supported by the support portion 135 to be movable between the plurality of preparation sections 144, the imaging section 145, and the therapy section 142 and may transport the patient 50 between the plurality of preparation sections 144, the imaging section 145, and the therapy section 142. In this case, the particle beam therapy device 250 can quickly check the fixation position of the patient 50 by performing the therapy simulation in a certain preparation section 144 to determine the fixation position, and then transporting the patient 50 to the imaging section 145 for imaging while keeping the patient 50 fixed. Additionally, since the imaging section 145 is provided near the preparation section, and the plurality of preparation sections 144 are provided for a single therapy section 142, the preparation room 104 can also be used as the simulation room. In this way, since there is a time margin, the preparation immediately before the therapy can be performed in the same preparation section 144 as the preparation section 144 where the simulation has been performed. In this case, since there is no need to correct alignment errors between the simulation and the actual therapy preparation, the preparation time immediately before the therapy can be shortened.

The particle beam therapy device 250 may further include the imaging room 105 that is independent of other rooms and that is provided with the imaging section 145, and the imaging room 105 may enable the imaging of the patient 50 prepared in each of the plurality of preparation rooms 104 that are each provided with the preparation section 144. In this case, the particle beam therapy device 250 can quickly check the fixation position of the patient 50 by performing the therapy simulation in a certain preparation room 104 to determine the fixation position, and then transporting the patient 50 to the imaging room 105 for imaging while keeping the patient 50 fixed. Additionally, since the imaging room 105 is provided near the preparation room 104, and the plurality of preparation rooms 104 are provided for a single therapy room 102, the preparation room 104 can also be used as the simulation room. In this way, since there is a time margin, the preparation immediately before the therapy can be performed in the same preparation room 104 as the preparation room 104 where the simulation has been performed. In this case, since there is no need to correct alignment errors between the simulation and the actual therapy preparation, the preparation time immediately before the therapy can be shortened.

In the particle beam therapy device 250, the transport mechanism 130 that transports the patient 50 between the therapy section 142 and the preparation sections 144 may transport a first patient 50 from a first preparation section 144 to the therapy section 142 and may transport, during the therapy of the first patient 50 in the therapy section 142, a second patient 50 from a second preparation section 144 to another room excluding the therapy section 142. In this case, it is possible to proceed with the therapy preparation for another second patient 50 in another preparation section 144 simultaneously with the therapy of the first patient 50.

In the particle beam therapy device 250, the transport mechanism 130 may transport the first patient 50 from the first preparation section 144 to the imaging section 145 for performing imaging of the patient 50 and may transport, during the imaging of the first patient 50 in the imaging section 145 or during the preparation for the first patient 50 in the first preparation section 144, the second patient 50 from the second preparation section 144 to the therapy section 142. In this case, it is possible to proceed with the therapy of another second patient 50 in another preparation section 144 simultaneously with the simulation of the first patient 50 or the imaging for checking the simulation.

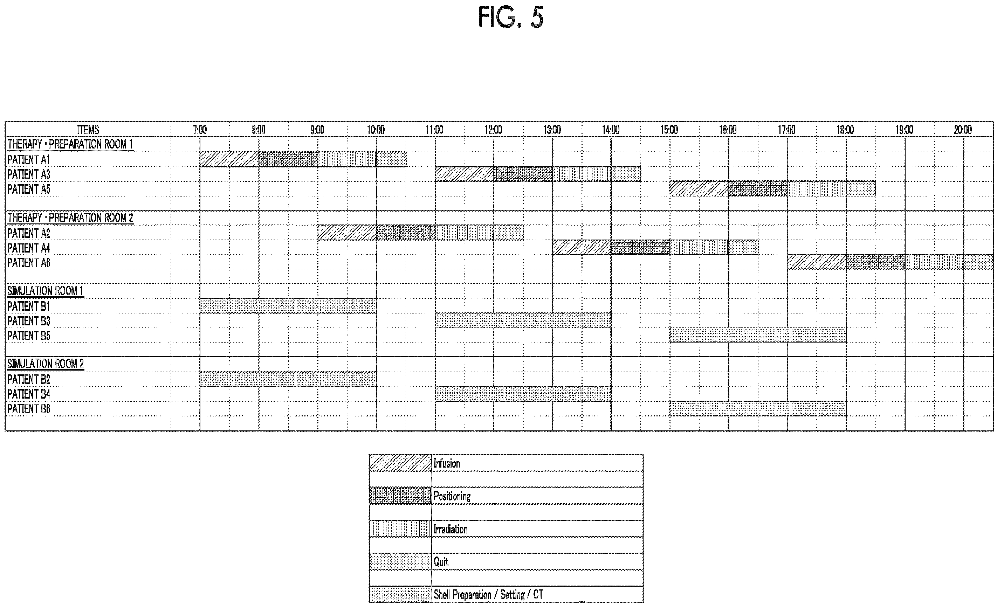

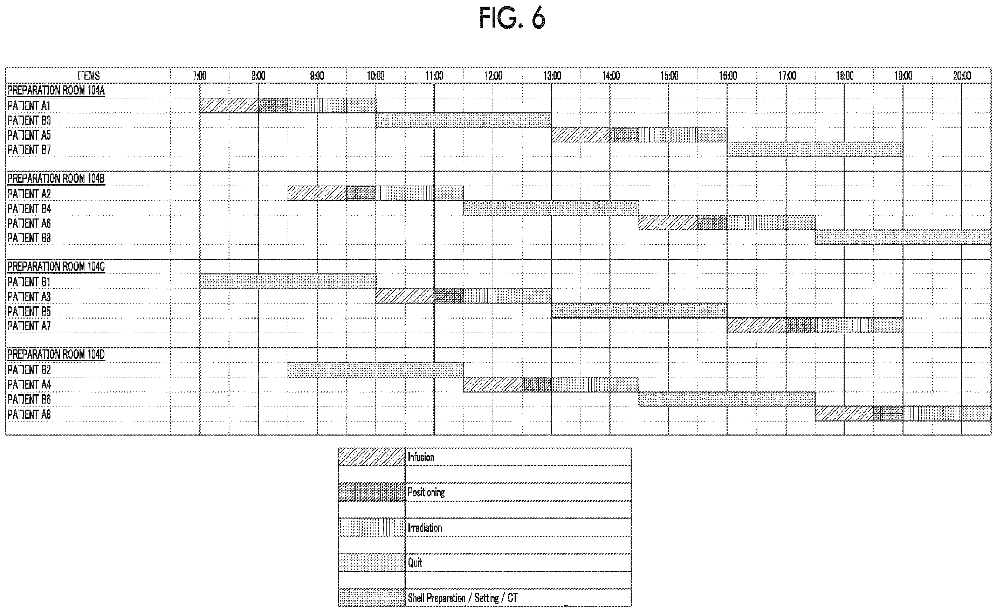

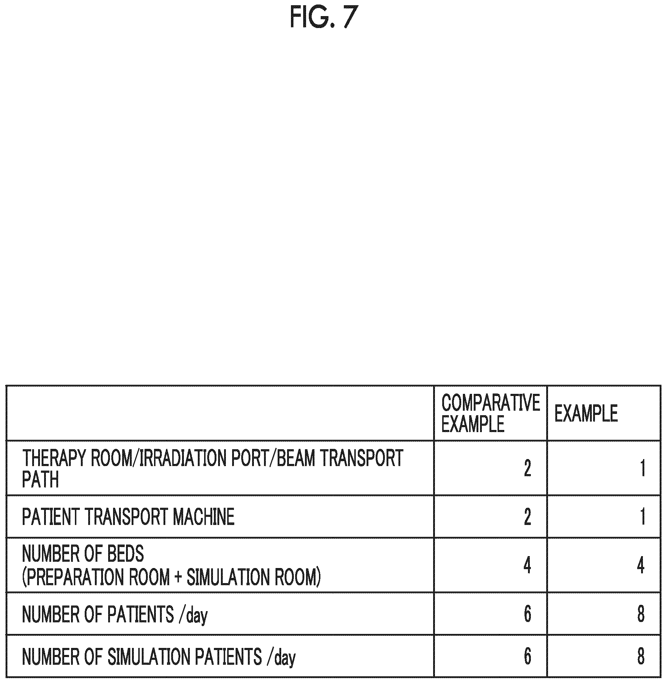

A comparison will be made between a comparative example shown in Fig. 5 and an example shown in Fig. 6. Fig. 7 shows conditions of the comparative example and the example. As shown in Fig. 7, a particle beam therapy facility according to the comparative example includes a therapy room, a preparation room, a simulation room, and a transport machine as one set, and includes two such sets. Fig. 5 shows the operation timing of the particle beam therapy facility 100 of the particle beam therapy facility according to the comparative example throughout a day. As shown in Figs. 5 and 7, in the comparative example, the number of patients that can undergo therapy in one day is six, and the number of patients that can undergo simulations is six. Fig. 6 is a diagram showing the operation timing of the particle beam therapy facility 100 according to the example and is a diagram showing the content of Fig. 4 in a manner that can be easily compared with Fig. 5. In the example, since the four preparation rooms can be used for both the preparation immediately before the therapy and the simulation, the waiting time per patient can be shortened. Additionally, since the preparation immediately before the therapy can be performed in the preparation room where the simulation has been performed, there is no need to correct alignment errors caused by using facilities in different rooms, and consequently, the "Positioning" time can be shortened as compared with the comparative example. Accordingly, as shown in Figs. 6 and 7, in the example, the number of patients that can undergo therapy in one day is eight, and the number of patients that can undergo simulations is eight, which is more than the number in the comparative example.

As described above, in the example, by providing the common transport mechanism 130 in the transfer room 103, it is not necessary to dispose the transport mechanism for each of the preparation rooms 104, which allows for a lower cost configuration of the facility. In addition, when one preparation room is provided for one therapy room as in the comparative example, it naturally becomes necessary to provide a plurality of therapy rooms. On the other hand, in the example, there is no need to provide an additional therapy room. That is, since there is no need to newly provide an additional irradiation port or an additional particle beam transport line to the irradiation port, significant reductions in devices and facility downsizing can be achieved, thereby resulting in lower introduction and maintenance costs.

The present disclosure is not limited to the above-described embodiment.

For example, the neutron generating device may use a nuclear reactor instead of an accelerator.

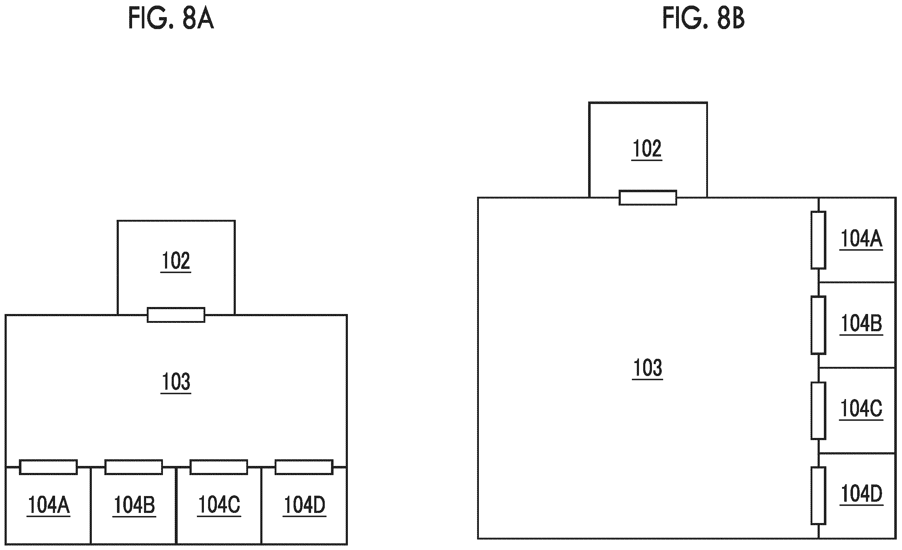

For example, the above-described configuration of the particle beam therapy facility is merely an example and can be appropriately modified. For example, as shown in Figs. 8A and 8B, layouts may be employed in which the preparation rooms 104 do not surround the transfer room.

### Reference Signs List

- 100: particle beam therapy facility
- 102: therapy room
- 103: transfer room
- 104: preparation room
- 105: imaging room
- 106: boron concentration measurement room
- 142: therapy section
- 144: preparation section
- 145: imaging section
- 130: transport mechanism
- 131: transport arm
- 135: support portion

## Claims

1. A particle beam therapy facility comprising:
a therapy room that is independent of other rooms and that is used to perform therapy with particle beams;
a plurality of preparation rooms that are used to perform preparations for the therapy; and
a transfer room that is independent of other rooms and that is used to transfer between the therapy room and the preparation rooms,
wherein the transfer room includes a single continuous space that allows communication between a therapy room entrance of the therapy room and a preparation room entrance of each of the plurality of preparation rooms.

2. The particle beam therapy facility according to claim 1, further comprising:
a support portion provided in the transfer room; and
a transport mechanism that is supported by the support portion to be movable between the plurality of preparation rooms and the therapy room and that transports a therapy target object between the plurality of preparation rooms and the therapy room.

3. The particle beam therapy facility according to claim 2,
wherein the transport mechanism is rotatable around a center axis extending in an up-down direction.

4. The particle beam therapy facility according to claim 1,
wherein the plurality of preparation rooms are disposed to surround the transfer room.

5. The particle beam therapy facility according to claim 1, further comprising:
a boron concentration measurement room that is independent of other rooms and that is used to measure a boron concentration of a therapy target object.

6. A particle beam therapy device comprising:
a support portion provided between a therapy section for performing therapy with particle beams and a plurality of preparation sections for performing preparations for the therapy; and
a transport mechanism that is supported by the support portion to be movable between the plurality of preparation sections and the therapy section and that transports a therapy target object between the plurality of preparation sections and the therapy section.

7. The particle beam therapy device according to claim 6, further comprising:
a therapy room that is independent of other rooms and that is provided with the therapy section;
a plurality of preparation rooms that are each provided with the preparation section; and
a transfer room that is independent of other rooms, that is provided with the transport mechanism, and that is used to transfer between the therapy room and the preparation rooms.

8. The particle beam therapy device according to claim 6,
wherein the plurality of preparation sections are disposed to surround the transport mechanism.

9. The particle beam therapy device according to claim 6,
wherein the transport mechanism includes a transport arm that is rotatable around a center axis extending in an up-down direction.

10. The particle beam therapy device according to claim 6,
wherein the support portion is provided between the therapy section, the plurality of preparation sections, and an imaging section for imaging the therapy target object, and
the transport mechanism is supported by the support portion to be movable between the plurality of preparation sections, the imaging section, and the therapy section and transports the therapy target object between the plurality of preparation sections and the imaging section, and the therapy section.

11. The particle beam therapy device according to claim 10, further comprising:
an imaging room that is independent of other rooms and that is provided with the imaging section,
wherein the imaging room enables imaging of the therapy target object prepared in each of the plurality of preparation rooms that are each provided with the preparation section.

12. The particle beam therapy facility according to claim 6,
wherein the transport mechanism
transports a first therapy target object from a first preparation section to the therapy section, and
transports, during therapy of the first therapy target object in the therapy section, a second therapy target object from a second preparation section to a plurality of other preparation sections excluding the therapy section or to an imaging section for imaging the therapy target object.

13. The particle beam therapy facility according to claim 6,
wherein the transport mechanism
transports a first therapy target object from a first preparation section to an imaging section for imaging the therapy target object, and
transports, during imaging of the first therapy target object in the imaging section or during preparation of the first therapy target object in the first preparation section, a second therapy target object from a second preparation section to the therapy section.
